(19) **Europäisches Patentamt**

**European Patent Office**

**Office européen des brevets**

(11) **EP 1 034 430 B1**

(12) **EUROPEAN PATENT SPECIFICATION**

(45) Date of publication and mention
of the grant of the patent:
**08.02.2006 Bulletin 2006/06**

(51) Int Cl.:
*G01N 33/543* *(2006.01)*      *G01G 3/16* *(2006.01)*
*G01R 29/22* *(2006.01)*      *G01N 27/00* *(2006.01)*

(21) Application number: **98957299.5**

(22) Date of filing: **24.11.1998**

(86) International application number:
**PCT/SE1998/002129**

(87) International publication number:
**WO 1999/030159 (17.06.1999 Gazette 1999/24)**

(54) **SENSOR FOR DETECTING BIOLOGICAL TARGET COMPLEXES**

SENSOR ZUR ERFASSUNG VON BIOLOGISCHEM KOMPLEX

CAPTEUR POUR DETECTION DE COMPLEXE CIBLE BIOLOGIQUE

(84) Designated Contracting States:
**AT BE CH CY DE DK ES FI FR GB GR IE IT LI LU
MC NL PT SE**

(30) Priority: **24.11.1997 SE 9704354**

(43) Date of publication of application:
**13.09.2000 Bulletin 2000/37**

(73) Proprietor: **Q-SENSE
412 96 Göteborg (SE)**

(72) Inventor: **HÖÖK, Fredrik
S-414 57 Göteborg (SE)**

(74) Representative: **Inger, Lars Ulf Bosson et al
Valea AB
Lindholmspiren 5
417 56 Gothenburg (SE)**

(56) References cited:
**WO-A1-96/35103**      **US-A- 5 653 939**

## Description

**Field of invention**

The present invention relates to a method for the measurement, using a piezo-electric crystal micro balance, of the interaction between at least one target molecule and at least one receptor molecule, of which at least one of the molecules is immobilised. The method includes measurement of dissipation (D) and frequency (f).

**Background of the invention**

Antibodies protect against diseases and infections. When a human being, for instance, is exposed to an antigen, the body produces antibodies with binding sites for the antigen, which thereby is immobilised. Accordingly, the antibodies can be used to decide whether a specific antigen is carried by a human being or not. The detection is performed by presenting the right antigen for a certain antibody and to decide whether binding takes place or not. The most developed analyses using immuno sensors include antigen radioactive labelling of the antigen/antibody complex or labelling with fluorescence. The most commonly used method using fluorescence is called ELISA (Enzyme Linked Immuno Sorbent Assay). ELISA is used among others to estimate if blood from a subject contains antibodies against a specific disease. Firstly the antigen from a virus or bacteria is bound to a surface. The surface is then exposed to the blood. If the blood contains antibodies specific to the antigen bound to the surface, they will bind to the antigen. If no antibodies are present, the surface will not be affected at all. At last the presence of antibodies are checked for by presenting the surface to a solution containing antibodies, labelled with a fluorescent group, which is specific for the antigen-antibody complex. If the surface contains the antigen-antibody complex, the labelled antibody will bind to the surface which can be detected. ELISA is a commonly used method and commercial systems and reagents are manufactured at a large scale, which renders the method relatively cheap. However, it presents several serious disadvantages. It is very time-consuming and its performance takes normally from a couple of hours up to a day. Monitoring in real time of the binding of the antibodies is not possible, i.e. no hints or information of the reaction kinetics of the system is being given.

The discovery of the linear relationship between the change in oscillator frequency in a piezo-electric crystal and change in mass on the crystal due to binding or adsorption, makes it possible to gravimetrically supervise the antigen-antibody reactions. The mathematical relationship between the change of frequency in a piezo-electric crystal, $\Delta f$, and the change of mass, $\Delta m$, at a crystal is given by the following formula:

$$\Delta f = -constant \cdot \Delta m.$$

It was for the first time reported in 1972, the usage of piezo-electric crystals for analytical purposes for antigen-antibody reactions (A. Shons, F. Dorman, J. Najarian, *J. Biomed. Mater. Res.* **6**:565). A crystal was covered with bovine serum albumine (BSA) and the forming of the BSA-antibody complex on the crystal was monitored by the change in frequency. Since then a number of studies of antigens and antibodies using piezo-electric methods has been performed. The development of this area has been concluded by A. A. Suleiman and G. G. Guilbault, *Analyst.*, **119**:2279 (1994) and M. D. Ward and D. A. Butty, *Science,* **249**:1000 (1990).

Several patents describe how QCM (abbreviation for Quartz Crystal Microbalance, i.e., a method of weighing using a quartz piezo-electric crystal) was used to analyse antigens and antibodies. In the American patent US-A-4,242,096, (1980), it is described how to determine the presence of an antigen in a sample by using adsorption of an antigen to a crystal. The antigen-coated crystal is exposed to a sample with a predetermined amount of antibody, whereupon the amount of antigen can be determined from the frequency shift, by using a standard plot.

In the American patent US-A-4,236,893 (1980) and US-A-4,314,821 (1982) to Rice methods are described wherein antibodies are detected using QCM. In the first mentioned patent, the antigen was immobilised on a crystal coated with a polymer. The frequency change from the binding of antibody was related to the concentration of antibody in the sample, but the analysis was disturbed by unspecific binding to the crystal. In the second patent, the detection of low molecular compounds is described using a crystal coated with anti-antibodies. All these analyses were performed by treating the crystal in solution and then measure the frequence in open air. This two-step procedure with solution /gas improves the sensibility with oscillating QCM, but causes technical complications and disturbs the hydration/dehydration which affects the frequency parameters.

Liquid phase piezo-electric immunoassay-technique has technical advantages as one can perform stationary analyses and flow analyses of a solution. Thus, the method has had physical limitations due to small frequency changes. Liquid phase piezo-electric-immunoassays has been reported by J. E. Roederer, G. J. Bastiaans, *Anal. Chem.*, **55:** 2333, (1983) and in their American patent US-A-4,735,906. The quartz crystal was here modified with glycidoxypropyl trimethoxysilane (GOPS) and the surface modified crystal was later additionally modified with anti-human IgG antibody and was then used for piezo-electric detection of human IgG. A similar method is described by H. Muramatu, J. M. Dicks, E. Tamiya and I. Karube, *Anal. Chem.,* **59**:2769 (1987), wherein the surface of quartz crystals was modified with γ-aminopropyl triethoxysilane and then with protein A. The surface modified crystal was then used to determine the concentration of human IgG.

In the PCT application WO97/04314 a method is described wherein the binding of e.g. antigen-antibody complex in solution is determined using a piezo-electric crystal. A weakness in this system and in the above mentioned liquid phase system is that separation of two closely related antibodies having e.g. different viscoelastic properties, is not possible since only the change in resonance frequency is measured.

The PCT application WO96/35103 describes an equipment for measurement of the resonance frequency and/or the dissipation factor in a piezo-electric resonance unit, wherein a piezo-electric crystal is connected to an operating unit to achieve oscillation of said crystal, at which oscillation was measured after switching off the crystal operating unit. The device can be used for the measurement of absorption of i.e. organic material to one surface of the crystal.

US-A-5,653,939 describes a method for investigation of a test sample with respect to target nucleic acids, the method comprising the formation of a set of test sites on several locations, each site comprising oligonucleotide probes formed there upon with known binding characteristics and wherein the probes at each test site differ from the probes at other test sites in a predetermined known way such that the localisation of the test sites of the probes and their binding characteristics also is known, that a test substance is applied on the test sites, and that a change in the electromagnetic properties is detected at the test sites, which arise from binding of target nucleic acid to the probes.

**Brief description of the invention**

The object of the present invention is to provide, using a piezo-electric crystal micro balance, a method for measurement of the interaction between at least one target molecule and at least one receptor molecule, of which at least one molecule is immobilised. The characterisation of the interaction between the target molecule and the receptor molecule includes the measurement of the dissipation (D) and/or measurement of the change in dissipation (ΔD) and the measurements of resonance frequency (f) and/or the change in resonance frequency (Δf).

In particular the invention relates to a method to measure the interaction between at least one target molecule and at least one receptor molecule using a piezo-electric crystal micro balance, where at least one type of the molecules is immobilised, which invention is characterized in that a receptor molecule is immobilised to a piezo-electric crystal micro balance, that at least one crystal is brought into oscillation by means of a driving circuit, that a target molecule is presented to a crystal micro balance surface comprising the immobilised receptor molecule, and permitting a reaction between the target molecule and the receptor molecule, and
that the dissipation (D) and/or the change in dissipation (ΔD) is measured during the driving and/or after switching off of the driving circuit,
that resonance frequency (f) and/or the change in resonance frequency (Δf) is measured during driving and/or after switching off of the driving circuit, as well.
whereby the quantities resonance frequency and the dissipation factor are measurements or signatures of the process on the surface of the crystal micro balance, which surface affects the resonance frequency and/or the dissipation factor.

In a preferred embodiment one or more, e.g., XY-diagram, so called Df-signature (-s) consisting of the quantities resonance frequency and the dissipation factor in the piezo-electric crystal micro balance, is used, with resonance frequency on one axis and the dissipation factor on the other axis.

In a preferred embodiment the target molecule and the receptor molecule are selected from one or more of the following groups comprising antibodies, synthetic antibodies, antibody fragment, antigens, synthetic antigens, haptenes, nucleic acids, synthetic nucleic acids, cells, receptors, hormones, proteins, prions, drugs, enzymes, carbohydrates, biotins, lectins, bacteria, virus and saccharides.

In a preferred embodiment the target molecule is an antibody and the receptor molecule is an antigen.

In a preferred embodiment the receptor molecule is selected in such manner that it is smaller than the target molecule and/or that the receptor molecule binds close to one end of the target molecule, as to create a large lever.

In a preferred embodiment the measurements are performed using equipment including at least one crystal with at least one set of electrodes connected to a driving circuit to bring the crystal/crystals in oscillation, which equipment is arranged to perform manual or automated measurements in real time of
the dissipation factor (D), and/or the change of this, (ΔD), and the resonance frequency (f) and/or the change of this, (Δf),
by driving and/or by disconnecting the drive circuit, and that the dissipation and the frequency is measured as a function of time.

In a preferred embodiment the piezo-electric crystal micro balance is of the QCM (Quartz Crystal Microbalance) type.

In a preferred embodiment the piezo-electric crystal micro balance/ balances are coated with a binding surface, which may include oxide, metal oxide, semi-conductor, inorganic compound, organic compound, lipid layer, polymer, avidine or strepavidine, biotin, protein and self organising layers.

In a preferred embodiment the measurements are performed in gas phase, vacuum or liquid phase.

In a preferred embodiment the measurements are performed at basic tone frequency as well as at a harmonic frequency and/or different oscillation amplitudes.

In a preferred embodiment the measurements are performed automatically as well as manually.

In a preferred embodiment the measurements are performed with different kinds of piezo-electric crystals, such as quartz, and different crystallographic cuts, such as AT- and BT-crystallographic cuts.

In a preferred embodiment the measurements of the resonance frequency and the dissipation of the piezo-electric crystal are performed as a function of time.

In a preferred embodiment the measurement is used to obtain a measure on the interaction of viscous and/or viscoelastic molecules, and/or their influence on the binding surface

In a preferred embodiment the amount of a certain target molecule, such as antibodies, antibody fragment, antigens, haptenes, nucleic acids, synthetic nucleic acids, cells, bacteria, receptors, hormones, proteins, prions, drugs, enzymes, carbohydrates, biotins, lectins, virus, metabolite (-s), vitamins and saccharides also consisting of other components, is determined, by equipping the surface with a receptor molecule.

In a preferred embodiment a sample flow is brought into contact with the detector, so that the amount of target molecule is determined as a function of time.

In a preferred embodiment the amount of nucleic acids, containing a certain sequence, is detected and/or determined in a sample, using a receptor molecule, which is a single-stranded nucleic acid having a sequence complementary to the molecule to be detected.

In a preferred embodiment point mutations are detected in a nucleic acid, by bringing a sample in simultaneous contact with two or more sensor surfaces equipped with receptor nucleic acid-strands, which are complementary to the mutated and the normal sequence respectively, and the difference of their response is determined.

The dissipation, according to the present invention, is a new quantity for the piezo-electric crystal micro balance measurements of target molecute-receptor molecule complexes. Previously only the resonance frequency has been determined and the present invention provides a method which enables measurement of both the dissipation and the resonance frequency.

The information of the change in dissipation and resonance frequency of a system can be related to the concentration of the target molecule in the solution and the affinity between the target molecule and the receptor molecule. This is possible as the different target complexes have viscoelastic properties, since the conformations of the complexes differ. In addition, the target complexes affect the absorbing layer differently, which also affect the result of the measurements. Accordingly, interaction between different target motecule-receptor molecule complexes according to the present invention, can be distinguished from their dissipation, or dissipations and frequency responses for a certain system. For instance, f and D are together said to form some kind of fingerprint of the measured system. The change in dissipation, or frequency and dissipation, when the target molecule interacts with the receptor molecule can then be used as a measurement and/or a signature of the process.

Use of the method according to the present invention requires:

1. a receptor molecule with a specificity for the target molecule;
2. a method for binding the receptor molecule to the piezo-electric crystal surface;
3. a suitable reaction conditions to form a reaction product such as target analyte receptor complex, and
4. an equipment for monitoring the change in dissipation and resonance frequency, or dissipation only.

With the expression interaction are all kinds of interaction appreciated which may be but not necessarily temporary and may comprise several molecules, and also the interaction of reaction products against the surface.

Method for binding the receptor molecule to the surface of the piezo-etectric crystal can either be performed directly via adhesion, or via binding surfaces.

The expression target molecule relates to compounds which are to be detected, which includes but are not limited to antigens, antibodies, drugs, nucleic acids, hormones, proteins, enzymes, and carbohydrates. The target molecule has to be able to bind to the receptor molecule, which also may be but are not limited to the above mentioned compounds.

The target molecule and the receptor molecule can form a target complex, e.g. antigen-antibody, ligand-receptor, sugar-lectin, biotin-avidine, enzyme-substrate, oligonucleotide-oligonucleotide with a complementary sequence, oligonucleotide-protein, oligonucleotide-cell, etc. The forming of these target complex can be detected, as well as the dissociation of such complexes.

The piezo-electric crystal micro balance is preferably of the QCM type, and are preferably used here by measurements in liquids but the measurements can also be performed in gas phase or in vacuum. The invention is a fast and sensitive technology for e.g. immunoreactions and in particular antibody-antigen reactions. Compared to other techniques for real time measurements of protein adsorption kinetics in liquid phase e.g., ellipsometry this technique is relatively cheap, as quartz crystals are also used as sensor chips in other application areas.

## Description of the drawings

Fig. 1 shows $\Delta f$ versus time for binding of the antigen gG-2 in concentrations of 1 $\mu$g /ml for two separate, but identically treated $TiO_2$-crystals;

Fig. 2 shows $\Delta D$ versus time for binding of the antigen gG-2 in concentrations of 1 $\mu$g/ml for two separate, but identically treated $TiO_2$-crystals;

Fig. 3 shows $\Delta f$ versus time for three separate measurements of a quartz crystal, which has been identically treated with $TiO_2$-gG-2-Hb, for binding of 0.5 mg /ml B9, G11, and B1;

Fig. 4 shows $\Delta D$ versus time for three separate measurements of a quartz crystal, which has been identically treated with $TiO_2$-gG-2-Hb, for binding of 0.5 mg /ml B9, G11, and B1;

Fig. 5 shows $\Delta D$ versus time for two separate measurements of a quartz crystal, which has been identically treated with $TiO_2$-gG-2-Hb, for binding of 0.5 mg /ml B9, G11, and B1.

## Detailed description of the invention

The present invention relates to a method to measure the interaction between at least one target molecule and at least one receptor molecule, using a piezo-electric crystal micro balance, preferably of the QCM type.

The Swedish patent of the applicant with publication number 504 199 describes a device and a technique for the measurement of resonance frequency and/or dissipation factor in a piezo-electric crystal micro balance, preferably of the QCM (Quartz Crystal Micro Balance) type. The present invention is a further development of the technique for a method to measure biological target complexes.

QCM is an extremely sensitive balance designed to weigh small quantities of material which are added or removed from the electrode. QCM according to the present invention consists of a crystal disc of a piezo-electric material, preferably quartz, more preferably a AT- or BT-cut quartz-crystal. Such a crystal can oscillate in shear mode with the amplitude of approximately 1-10 nm and with the basic tone frequency

$$f = constant \cdot d^{-1}$$

wherein d is the thickness of the crystal. With d $\approx$ 0.17 is f $\approx$ 10 MHz. The oscillation occurs when a AC field is brought perpendicular to the surface of the crystal. The frequency of the AC-field should be centred around the natural frequency of the crystal. In practice, the electrodes are applied by e.g. vaporisation on each side of the crystal. The electrodes are then in electric contact with an external oscillation circuit. This device can measure very small changes in mass on the quartz electrodes, in the range of 1 ng /$cm^2$, or even less, under advantageous conditions.

Under ideal conditions, as been mentioned above, the change in resonance frequency, $\Delta f$, is proportional against the change in mass, $\Delta m$, i.e.

$$\Delta f = -constant \cdot \Delta m$$

The size of the constant depends on the choice of crystal and cut thereof. The equation presumes that the mass, which is represented as $\Delta m$, is rigidly located, is evenly distributed on the electrode and follows the oscillation of QCM without dissipative losses. The equation is not generally valid when the coated mass consists of a viscous material and/or is not equally distributed over the mass sensitive area of the crystal. In that case a given amount of coated mass can also give rise to different frequency shifts, which is the same as that a certain frequency shift can correspond to different amounts of mass. Thus in these cases, it can be of limited value only to measure the resonance frequency.

The object of the present invention is to construct a sensitive method for the detection of biological target complexes, using an improved QCM-technique. This can be achieved by an improved characterisation of the system being measured by the sensor. By simultaneously measuring f and the dissipation factor D, (the dissipation being the inverse of the so called Q-factor) of the QCM, the amount and the value of the information in the studied system is significantly increased. A typical measuring method according to the present invention is to both measure f and D, as a function of time. The quantities f and D measure different features of the system. Together f and D provide a kind of finger print of the studied system. However, in certain systems, the measurement of the dissipation, D, is enough.

The method according to the present invention allows system-specific finger prints.

Processes/system are being characterised by plotting each measured $\Delta f$ versus $\Delta D$ in a XY-graph, or in any coordinate system, with $\Delta f$ on one axis and $\Delta D$ on the other. The obtained plot, the so called Df-plot, is not explicit, but implicit, time dependent since each pair of $\Delta f$- $\Delta D$ can be put in the plot. The shape of the Df plot works as a finger print for a given process.

Further finger prints can be obtained by exciting the crystal in its harmonic tones (3rd, 5th etc.) and monitor the Df-plot for every harmonic tone. It is also possible to use different cuts of the crystal simultaneously, not only the AT-cut, and monitor the Df-plot for its different cuts. Further the Df-plot can be measured at different oscillation amplitudes.

That which is called the Df-finger print or Df-signature of a system, according to the invention includes all the Df-plots, which can be measured for the studied /measured system at different oscillation frequencies, different oscillation modes and different amplitudes.

The shape of the Df-plot can immediately reveal that the interaction between different types of the target molecule-receptor molecule complex is different, without a detailed interpretation of the nature of the interaction having to be known. Each shift or layer, for e.g., non-rigid and viscous layers on the surface of the crystal has accordingly an Df-signature each. If the signature of two systems at one frequency and/or amplitude are too similar, the difference may increase at other frequencies and amplitudes.

Requirements for using the method according to the present invention:

1. A receptor molecule having a specificity for the target molecule

The receptor can be any of the following compounds: antibodies, synthetic antibodies, antibody fragment, antigens, synthetic antigens, haptenes, nucleic acids, synthetic nucleic acids, cor, receptors, hormones, proteins, prions, drugs, enzymes, carbonhydrates, biotins, lectins, bacteria, virus and saccharides, The only requirement present on the receptor molecule is must be able to bind to the target molecule, which can be any of the above-mentioned compounds as well. The target molecule and the receptor molecule form a target complex, e.g., antigen-antibody, ligand-receptor, sugar-lectin, biotin-avidine, enzyme-substrate, oligonucleotide-oligonucleotide with a complementary sequence, oligonucleotide-protein, oligonucleotide-cell, etc. The receptor molecule can also be selected in such way that it is smaller than the target molecule and/or that the receptor molecule binds close to one end at the target molecule to create a large lever.

2. Method for binding the receptor molecule to the piezo-electric crystal surface

The receptor molecule can be attached directly to the piezo-electric crystal surface through adhesion or via another surface with or without yet another layer, together called the binding surface, through adhesion or a chemical reaction. The binding surface may include oxide, metal oxide, such as $TiO_2$ and $SiO_2$, semi-conductors, inorganic compound, organic compound, lipid layer, polymer, avidine or streptavidine, biotin, protein, and self organised layers. The choice of binding surface may affect the finger print, that is, some surfaces, e.g., $TiO_2$ may amplify the signal of the analysis.

3. Suitable reaction conditions to form a reaction product, such as the target analyte receptor complex

These are all dependant on which system to study, and it is known in the art which reaction conditions are suitable for a certain system. Examples of suitable reaction conditions are, but are not limited to, from 4 °C till 80 °C, pH 4 - 9.0, a salt concentration between 1 $\mu$M - 1 M.

4. Equipment to monitor the change in dissipation and resonance freauency, or dissipation only.

The measurement are performed using equipment including at least one crystal with at least one set of electrodes (applied directly on the crystal or in close connection to this) connected to a drive circuit, to put the crystal/crystals in oscillation, which is arranged to perform a manual or automatised measurement in real time of

(i) the dissipation factor (D), and/or the change of this, ($\Delta$D), or
(ii) the dissipation factor(D), and/or the change of this, ($\Delta$D), and the resonance frequency (f) and/or the change of this ($\Delta$f),

by driving and/or by disconnecting the drive circuit, and the measurement of the dissipation, or the dissipation and the frequency is measured as a function of time.

The measurements may be performed in gas phase, vacuum, but preferably in liquid phase. The measurements can be performed at basic tone frequency as well as harmonic tones, and/or the oscillation amplitude and it can be performed automatically as well as manually.

To reduce unspecific binding of the target analyte a blocking agent can be used. Then it covers the areas or parts of areas, on the crystal surface or at the binding surface which are not coated with receptor molecules. Several proteins or other molecules can be used as blocking agent. However, they should have a low, or lowest possible affinity to the target molecule.

Measurement on a sample flow can also be performed, which is brought in contact with the detector, so that the content of target molecule is determined as a function of time.

It need not have to be the binding course itself, which is the main object, which is being studied, but the product, which is the result of the binding. Measurements can also be performed in which way the target complex affects the binding surface and how viscous and/or viscoelastic molecules interact. In addition, measurements of the dissociation of a target complex can also be performed, e.g., an antigen-antibody complex, which dissociates due to effects from the outside.

The method according to the present invention can also be used to detect and/or determine the amount of nucleic acids of a certain sequence present in a sample, using a receptor molecule, which is a single-stranded nucleic acid having a complementary sequence to the target molecule, which are to be detected. Additionally, point mutations in the nucleic acid can be detected by bringing a sample into simultaneous contact with two, or more sensor surfaces, equipped with receptor nucleotide strands, which are complementary to the mutated and the normal sequence, respectively, and measure the difference in their responses.

Below a system is given as an example, wherein the target molecule is an antibody and the receptor molecule is an antigen. The QCM technology, according to the present invention hereby allows a rapid (less than one hour) real time measurement of the antibody-antigen reaction. It is done by measurement of the dissipation and the frequency shift during time, as the antibodies binds to the antigen which covers the crystal surface. In addition, the kinetics of the antibody-antigen reaction can be measured.

Using the method according to the present invention the reaction kinetics can be directly related to

(i) the antibody concentration in the solution, and
(ii) the affinity between antigen and antibody.

These two parameters is of great importance for, e.g., medical analyses.

Example 1

The receptor molecule was the antigen glycoprotein G (gG-2) from Herpes Simplex virus type 2 (HSV-2). This protein, having a molecular weight of 120 kD, was purified from virus infected cell membranes using helix promatia lectin affinity chromatography. The invention was demonstrated using three different target molecules, the monoclonal $\gamma$-globulin (IgG) the antibodies B9, G11, and B1. It is known that the antibodies B9 and G11 are type-specific and do not bind to other Herpes Simplex virus proteins than HSV-2. Using the ELISA technique it has been proved that these antibodies are specific to two different epitopes on gG-2 (Liljequist, J. Å., Tribala, E., Svennerholm, B., Jansson, S., Sjögren-Jansson, E., Bergström, T., submitted to Journal of General Virology 1997). The binding affinity to the antigen is approximately 100 times larger for B9 than for G 11. The monoclonal antibody B1 which is type-specific for glycoprotein C from Herpes Simplex type 1,were used as control, as it is known that it does not bind to gG-2.

Titan oxide ($TiO_2$) was used as a substrate surface. Between the reactions the substrate surface was washed several times. It could therefore be used up to 10 times with a high reproducibility.

At the binding of the antigen to the surface, a 50 mM TRIS/ 200 mM KCl buffer with pH 9.6 was used, and for the binding of antibody a 50 mM HEPES/ 200 mM KCl buffer with pH 7.0 was used.

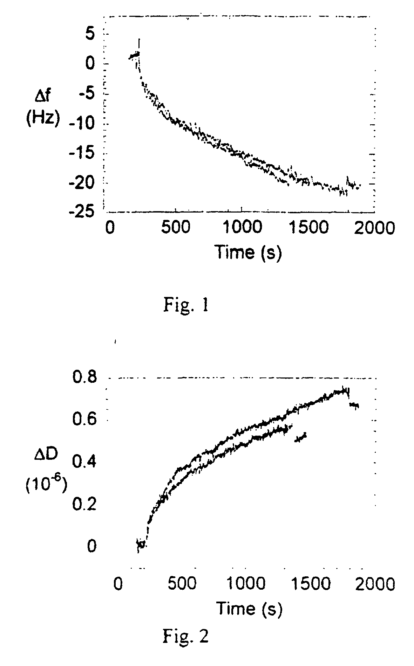

Figure 1 shows $\Delta f$ versus time and Figure 2 shows $\Delta D$ versus the time for binding of the antigen gG-2 at a concentration 1 $\mu$g / ml for two separate, but identically treated $TiO_2$-crystals. The antigen was hereby immobilised before the detection of the antibody. The adsorption did not reach saturation during the measurements, but the binding of the antigen was interrupted when the frequency reached approximately 20 Hz. Measurements were performed at the third harmonic tone. The binding of antigen to different crystals were performed with a high reproducibility, which is illustrated in Figure 1 and 2. To avoid unspecific binding of antibodies to free surface 30 $\mu$g /ml hemoglobin (Hb) was used, as a blocking agent (bovine hemoglobin, Sigma). Thereafter the gG-2-Hb surface was exposed to the antibody solution to be examined.

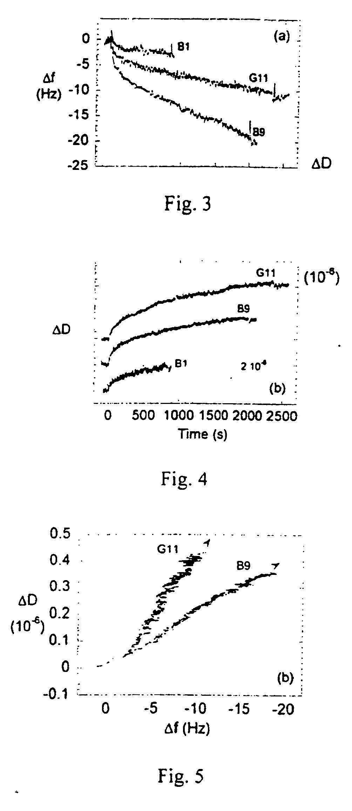

Figure 3 and 4 show $\Delta f$ versus time and $\Delta D$ versus time of three separate measurements with identically treated $TiO_2$-gG-2-Hb crystals for binding of 0.5 mg/ml B9, G11 and B1.

The control antibody, B1, gave a very small change in f and D, which shows that the unspecific binding was low. Corresponding shifts in f and D for B9 and G11 are significantly higher. It can also be seen that the antibody B9 having a higher affinity than G11, binds at a higher rate and gives a two times higher frequency change than for G11, but without any greater change in D-shift.

The results shown in Figure 5, wherein $\Delta D$ versus $\Delta f$ is given of the binding of B9 and G11. Both B9 and G11 exhibit linearity between $\Delta f$ and $\Delta D$, but the slope for G11 is approximately 1.7 times bigger than for B9. It should also be noted that the slopes, i.e. $\Delta D/\Delta f$, were identical for respective antibody at both low and high concentrations. This shows that antibodies specific for different epitopes on the same antigen can be distinguished from each other, not from velocity or absolute frequency shift for the reaction alone, but also from induced $\Delta D$ per $\Delta f$. The finger print obtained from Df-signature makes it possible to distinguish between antibodies specific for different epitopes, but having similar affinities. This depends on the fact that the observed changes in dissipation are caused by the different conformations, created by different antibody-epitope reactions by the antigen-antibody complexes. In addition, the different antigen-antibody complexes affect the absorbed layer differently.

Consequently different antibody-antigen reactions can be separated, according to the present invention, in a unique way differentiated from their dissipation and frequency answer. This is of great importance since antibodies from different bacteria and virus infections can be detected with a high sensibility and specificity.

Claims

1. Method to measure the interaction between at least one target molecule and at least one receptor molecule using a piezo-electric crystal micro balance, where at least one type of the molecules is immobilised,
   **characterised in,**
   **that** a receptor molecule is immobilised to a piezo-electric crystal micro balance, that at least one crystal is brought into oscillation by means of a driving circuit, that a target molecule is presented to a crystal micro balance surface comprising the immobilised receptor molecule, and permitting a reaction between the target molecule and the receptor molecule, and

**that** the dissipation (D) and/or the change in dissipation (ΔD) is measured during the driving and/or after switching off of the driving circuit,

**that** resonance frequency (f) and/or the change in resonance frequency (Δf) is measured during driving and/or after switching off of the driving circuit, as well. whereby the quantities resonance frequency and the dissipation factor are measurements or signatures of the process on the surface of the crystal micro balance, which surface affects the resonance frequency and/or the dissipation factor.

2. Method according to claim 1,
   **characterised in,**
   **that** one or more, e.g., XY-diagram, so called Df-signature (-s) consisting of the quantities resonance frequency and the dissipation factor in the piezo-electric crystal micro balance, is used, with resonance frequency on one axis and the dissipation factor on the other axis.

3. Method according to one or more of the preceding claims,
   **characterised in,**
   **that** the target molecule and the receptor molecule are selected from one or more of the following groups comprising antibodies, synthetic antibodies, antibody fragment, antigens, synthetic antigens, haptenes, nucleic acids, synthetic nucleic acids, cells, receptors, hormones, proteins, prions, drugs, enzymes, carbohydrates, biotins, lectins, bacteria, virus and saccharides.

4. Method according to claim 3,
   **characterised in,**
   **that** the target molecule is an antibody and the receptor molecule is an antigen.

5. Method according to one or more of the preceding claims,
   **characterised in,**
   **that** the receptor molecule is selected in such manner that it is smaller than the target molecule and/or that the receptor molecule binds close to one end of the target molecule, as to create a large lever.

6. Method according to one or more of the preceding claims,
   **characterised in,**
   **that** the measurements are performed using equipment including at least one crystal with at least one set of electrodes connected to a driving circuit to bring the crystal/crystals in oscillation, which equipment is arranged to perform manual or automated measurements in real time of

the dissipation factor (D), and/or the change of this, (ΔD), and the resonance frequency (f) and/or the change of this, (Δf),

by driving and/or by disconnecting the drive circuit, and that the dissipation and the frequency is measured as a function of time.

7. Method according to one or more of the preceding claims,
   **characterised in,**
   **that** the piezo-electric crystal micro balance is of the QCM (Quartz Crystal Microbalance) type.

8. Method according to one or more of the preceding claims,
   **characterised in,**
   **that** the piezo-electric crystal micro balance/ balances are coated with a binding surface, which may include oxide, metal oxide, semi-conductor, inorganic compound, organic compound, lipid layer, polymer, avidine or strepavidine, biotin, protein and self organising layers.

9. Method according to one or more of the preceding claims,
   **characterised in,**
   **that** the measurements are performed in gas phase, vacuum or liquid phase.

10. Method according to one or more of the preceding claims,
    **characterised in,**
    **that** the measurements are performed at basic tone frequency as well as at a harmonic frequency and/or different oscillation amplitudes.

11. Method according to one or more of the preceding claims,
    **characterised in,**
    **that** the measurements are performed automatically as well as manually.

12. Method according to one or more of the preceding claims,
    **characterised in,**
    **that** the measurements are performed with different kinds of piezo-electric crystals, such as quartz, and different crystallographic cuts, such as AT- and BT-crystallographic cuts.

13. Method according to one or more of the preceding claims,
    **characterised in,**
    **that** the measurements of the resonance frequency and the dissipation of the piezo-electric crystal are performed as a function of time.

14. Method according to one or more of the preceding claims,
**characterised in,**
**that** the measurement is used to obtain a measure on the interaction of viscous and/or viscoelastic molecules, and/or their influence on the binding surface

15. Method according to one or more of the preceding claims,
**characterised in,**
**that** the amount of a certain target molecule, such as antibodies, antibody fragment, antigens, haptenes, nucleic acids, synthetic nucleic acids, cells, bacteria, receptors, hormones, proteins, prions, drugs, enzymes, carbohydrates, biotins, lectins, virus, metabolite (-s), vitamins and saccharides also consisting of other components, is determined, by equipping the surface with a receptor molecule.

16. Method according to one or more of the preceding claims,
**characterised in,**
**that** a sample flow is brought into contact with the detector, so that the amount of target molecule is determined as a function of time.

17. Method according to one or more of the preceding claims,
**characterised in,**
**that** the amount of nucleic acids, containing a certain sequence, is detected and/or determined in a sample, using a receptor molecule, which is a single-stranded nucleic acid having a sequence complementary to the molecule to be detected.

18. Method according to one or more of the preceding claims,
**characterised in,**
**that** point mutations are detected in a nucleic acid, by bringing a sample in simultaneous contact with two or more sensor surfaces equipped with receptor nucleic acid-strands, which are complementary to the mutated and the normal sequence respectively, and the difference of their response is determined.

**Patentansprüche**

1. Verfahren zur Messung der Wechselwirkung zwischen wenigstens einem Targetmolekül und wenigstens einem Rezeptormolekül unter Verwendung einer piezoelektrischen Kristall-Mikrobalance, wobei wenigstens ein Typ der Moleküle immobilisiert ist,
**dadurch gekennzeichnet,**
**daß** ein Rezeptormolekül zu einer piezoelektrischen Kristall-Mikrobalance immobilisiert ist,
**daß** wenigstens ein Kristall mittels eines Treiberkreises zur Schwingung gebracht wird,
**daß** ein Targetmolekül einer Kristall-Mikrobalance-Oberfläche präsentiert wird, die das immobilisierte Rezeptormolekül umfaßt, und eine Reaktion zwischen dem Targetmolekül und dem Rezeptormolekül zulassen wird, und
**daß** die Dissipation (D) und/oder die Änderung der Dissipation (ΔD) während des Treibens und/oder nach dem Abschalten des Treiberkreises gemessen wird,
**daß** die Resonanzfrequenz (f) und/oder die Änderung der Resonanzfrequenz (Δf) während des Treibens und/oder nach dem Abschalten des Treiberkreises ebenfalls gemessen wird,
wodurch die Größen Resonanzfrequenz und der Dissipationsfaktor Messungen oder Signaturen des Prozesses auf der Oberfläche der Kristall-Mikrobalance sind, welche Oberfläche die Resonanzfrequenz und/oder den Dissipationsfaktor beeinflußt.

2. Verfahren nach Anspruch 1,
**dadurch gekennzeichnet,**
**daß** eine oder mehrere, z.B. XY-Diagamm, sogenannte Df-Signatur(en), bestehend aus den Größen Resonanzfrequenz und dem Dissipationsfaktor in der piezoelektrischen Kristall-Mikrobalance verwendet wird mit der Resonanzfrequenz auf der einen Achse und dem Dissipationsfaktor auf der anderen Achse.

3. Verfahren nach einem oder mehreren der vorhergehenden Ansprüche,
**dadurch gekennzeichnet,**
**daß** das Targetmolekül und das Rezeptormolekül aus einer oder mehreren der folgenden Gruppen, umfassend Antikörper, synthetische Antikörper, Antikörperfragment, Antigene, synthetische Antigene, Haptene, Nukleinsäuren, synthetische Nukleinsäuren, Zellen, Rezeptoren, Hormone, Proteine, Prionen, Arzneimittel, Enzyme, Kohlehydrate, Biotine, Lektine, Bakterien, Viren und Sacchariden, gewählt werden.

4. Verfahren nach Anspruch 3,
**dadurch gekennzeichnet,**
**daß** das Targetmolekül ein Antikörper und das Rezeptormolekül ein Antigen ist.

5. Verfahren nach einem oder mehreren der vorhergehenden Ansprüche,
**dadurch gekennzeichnet,**
**daß** das Rezeptormolekül auf solche Weise ausgewählt wird, daß es kleiner als das Targetmolekül ist, und/oder daß sich das Rezeptormolekül nahe einem Ende des Targetmoleküls bindet, um einen großen Hebel zu bilden.

6. Verfahren nach einem oder mehreren der vorhergehenden Ansprüche,
**dadurch gekennzeichnet,**

**daß** die Messungen unter Verwendung von Ausstattung ausgeführt werden, umfassend wenigstens ein Kristall mit wenigstens einem Satz von Elektroden, die mit einem Treiberkreis verbunden sind, um den (die) Kristall(e) in Schwingung zu versetzen, welche Ausstattung so angeordnet ist, daß sie in Echtzeit manuelle oder automatisierte Messungen des Dissipationsfaktors (D) und/oder der Änderung derselben (ΔD) und der Resonanzfrequenz (f) und/oder der Änderung derselben (Δf) ausführt, durch Treiben und/oder Abschalten des Treiberkreises, und daß die Dissipation und die Frequenz abhängig von der Zeit gemessen wird.

7. Verfahren nach einem oder mehreren der vorhergehenden Ansprüche,
   **dadurch gekennzeichnet,**
   **daß** die piezoelektrische Kristall-Mikrobalance vom QCM (Quarzkristall-Mikrobalance) -Typ ist.

8. Verfahren nach einem oder mehreren der vorhergehenden Ansprüche,
   **dadurch gekennzeichnet,**
   **daß** die piezoelektrische Kristall-Mikrobalance/Balancen mit einer Bindeoberfläche überzogen sind, welche Oxid, Metalloxid, Halbleiter, anorganische Zusammensetzung, organische Zusammensetzung, Lipidschicht, Polymer, Avidin oder Strepavidin, Biotin, Protein und selbstorganisierende Schichten enthalten kann.

9. Verfahren nach einem oder mehreren der vorhergehenden Ansprüche,
   **dadurch gekennzeichnet,**
   **daß** die Messungen in Gasphase, Vakuum oder flüssiger Phase ausgeführt werden.

10. Verfahren nach einem oder mehreren der vorhergehenden Ansprüche,
    **dadurch gekennzeichnet,**
    **daß** die Messungen bei Basistonfrequenz sowie bei einer harmonischen Frequenz und/oder verschiedenen schwingungsamplituden ausgeführt werden.

11. Verfahren nach einem oder mehreren der vorhergehenden Ansprüche,
    **dadurch gekennzeichnet,**
    **daß** die Messungen automatisch sowie manuell ausgeführt werden.

12. Verfahren nach einem oder mehreren der vorhergehenden Ansprüche,
    **dadurch gekennzeichnet,**
    **daß** die Messungen mit unterschiedlichen Arten von piezoelektrischen Kristallen, beispielsweise Quarz, und verschiedenen kristallographischen Schnitten, beispielsweise kristallographischen AT- und BT-Schnitten, ausgeführt werden.

13. Verfahren nach einem oder mehreren der vorhergehenden Ansprüche,
    **dadurch gekennzeichnet,**
    **daß** die Messungen der Resonanzfrequenz und der Dissipation des piezoelektrischen Kristalls abhängig von der Zeit ausgeführt werden.

14. Verfahren nach einem oder mehreren der vorhergehenden Ansprüche,
    **dadurch gekennzeichnet,**
    **daß** die Messung verwendet wird, um eine Messung der Wechselwirkung von viskosen und/oder viskoelastischen Molekülen und/oder deren Einfluß auf die Bindefläche zu erhalten.

15. Verfahren nach einem oder mehreren der vorhergehenden Ansprüche,
    **dadurch gekennzeichnet,**
    **daß** die Menge eines bestimmten Targetmoleküls, beispielsweise von Antikörpern, Antikörperfragment, Antigenen, Haptenen, Nukleinsäuren, synthetischen Nukleinsäuren, Zellen, Bakterien, Rezeptoren, Hormonen, Proteinen, Prionen, Arzneimittel, Enzymen, Kohlehydraten, Biotinen, Lektinen, Viren, Metabolit(en), Vitaminen und Sacchariden, auch aus anderen Komponenten bestehend, bestimmt wird, indem die Fläche mit einem Rezeptormolekül ausgestattet wird.

16. Verfahren nach einem oder mehreren der vorhergehenden Ansprüche,
    **dadurch gekennzeichnet,**
    **daß** eine Probenströmung in Kontakt mit dem Detektor gebracht wird, so daß die Menge des Targetmoleküls abhängig von der Zeit bestimmt wird.

17. Verfahren nach einem oder mehreren der vorhergehenden Ansprüche,
    **dadurch gekennzeichnet,**
    **daß** die Menge der Nukleinsäuren, die eine bestimmte Sequenz enthalten, detektiert wird, und/oder in einer Probe unter Verwendung eines Rezeptormoleküls bestimmt wird, welches eine Einstrang-Nukleinsäure mit einer Sequenz komplentär zu dem zu detektierenden Molekül ist.

18. Verfahren nach einem oder mehreren der vorhergehenden Ansprüche,
    **dadurch gekennzeichnet,**
    **daß** Punktmutationen in einer Nukleinsäure detektiert werden, indem eine Probe in gleichzeitigen Kontakt mit zwei oder mehr Sensorflächen gebracht wird, die mit Rezeptor-Nukleinsäure-Strängen ausgestattet sind, welche komplementär zu der mutierten bzw. der normalen Sequenz sind, und die Differenz ihres Ansprechens bestimmt wird.

## Revendications

1. Méthode de mesure de l'interaction entre au moins une molécule cible et au moins une molécule réceptrice en utilisant une microbalance à cristal piézo-électrique où au moins un type des molécules est immobilisé,
   **caractérisée en ce que**
   une molécule réceptrice est immobilisée sur une microbalance à cristal piézo-électrique, au moins un cristal est mis en oscillation au moyen d'un circuit de commande, une molécule cible est présentée sur une surface de microbalance cristalline comprenant la molécule réceptrice immobilisée et permettant une réaction entre la molécule cible et la molécule réceptrice, et **en ce que**
   on mesure la dissipation (D) et/ou la variation de la dissipation (ΔD) lors de la commande et/ou après l'arrêt du circuit de commande,
   on mesure également la fréquence de résonance (f) et/ou la variation de la fréquence de résonance (Δf) lors de la commande et/ou après l'arrêt du circuit de commande,
   les grandeurs fréquence de résonance et facteur de dissipation étant des mesures ou des signatures du processus qui se déroule sur la surface de la microbalance cristalline, ladite surface affectant la fréquence de résonance et/ou le facteur de dissipation.

2. Méthode selon la revendication 1,
   **caractérisée en ce que**
   on utilise une ou plusieurs signatures dites Df, par exemple un diagramme XY, constituées par les grandeurs fréquence de résonance et facteur de dissipation dans la microbalance à cristal piézo-électrique avec la fréquence de résonance sur un axe et le facteur de dissipation sur l'autre axe.

3. Méthode selon l'une au moins des revendications précédentes,
   **caractérisée en ce que**
   la molécule cible et la molécule réceptrice sont choisies dans un ou plusieurs des groupes suivants, comprenant des anticorps, des anticorps synthétiques, des fragments d'anticorps, des antigènes, des antigènes synthétiques, des haptènes, des acides nucléiques, des acides nucléiques synthétiques, des cellules, des récepteurs, des hormones, des protéines, des prions, des médicaments, des enzymes, des hydrates de carbone, des biotines, des lectines, des bactéries, des virus et des saccharides.

4. Méthode selon la revendication 3,
   **caractérisée en ce que**
   la molécule cible est un anticorps et la molécule réceptrice est un antigène.

5. Méthode selon l'une au moins des revendications précédentes,
   **caractérisée en ce que**
   la molécule réceptrice est choisie de telle façon qu'elle est plus petite que la molécule cible et/ou que la molécule réceptrice se lie à proximité d'une extrémité de la molécule cible de façon à créer un grand levier.

6. Méthode selon l'une au moins des revendications précédentes,
   **caractérisée en ce que**
   les mesures se font en utilisant un équipement comprenant au moins un cristal avec au moins un ensemble d'électrodes relié à un circuit de commande pour mettre le ou les cristaux en oscillation, l'équipement étant agencé pour effectuer des mesures manuelles ou automatisées en temps réel du facteur de dissipation (D) et/ou de sa variation (ΔD) et de la fréquence de résonance (f) et/ou de sa variation (Δf), en commandant et/ou en déconnectant le circuit de commande, et **en ce que** la dissipation et la fréquence sont mesurées en fonction du temps.

7. Méthode selon l'une au moins des revendications précédentes,
   **caractérisée en ce que**
   la microbalance à cristal piézo-électrique est du type QCM (*Quartz Cristal Microbalance :* microbalance à cristal de quartz).

8. Méthode selon l'une au moins des revendications précédentes,
   **caractérisée en ce que**
   la ou les microbalances à cristal piézo-électrique sont recouvertes avec une surface de liaison qui peut comprendre un oxyde, un oxyde métallique, un semi-conducteur, un composé inorganique, un composé organique, une couche lipidique, un polymère, de l'avidine ou de la streptavidine, de la biotine, une protéine et des couches auto-organisées.

9. Méthode selon l'une au moins des revendications précédentes,
   **caractérisée en ce que**
   les mesures se font en phase gazeuse, sous vide ou en phase liquide.

10. Méthode selon l'une au moins des revendications précédentes,
    **caractérisée en ce que**
    les mesures se font à une fréquence vocale fondamentale ainsi qu'à une fréquence harmonique et/ou avec des amplitudes d'oscillation différentes.

11. Méthode selon l'une au moins des revendications précédentes,
    **caractérisée en ce que**
    les mesures se font automatiquement de même que manuellement.

**12.** Méthode selon l'une au moins des revendications précédentes,
**caractérisée en ce que**
les mesures se font avec des types de cristaux piézo-électriques différents, par exemple le quartz et des coupes cristallographiques différentes, par exemple des coupes cristallographiques AT et BT.

**13.** Méthode selon l'une au moins des revendications précédentes,
**caractérisée en ce que**
les mesures de la fréquence de résonance et de la dissipation du cristal piézo-électrique se font en fonction du temps.

**14.** Méthode selon l'une au moins des revendications précédentes,
**caractérisée en ce que**
on utilise la mesure pour obtenir une mesure de l'interaction de molécules visqueuses et/ou visco-élastiques et/ou de leur influence sur la surface de liaison.

**15.** Méthode selon l'une au moins des revendications précédentes,
**caractérisée en ce que**
la quantité d'une certaine molécule cible, telle que des anticorps, des fragments d'anticorps, des antigènes, des haptènes, des acides nucléiques, des acides nucléiques synthétiques, des cellules, des bactéries, des récepteurs, des hormones, des protéines, des prions, des médicaments, des enzymes, des hydrates de carbone, des biotines, des lectines, des virus, un ou des métabolites, des vitamines et des saccharides également constitués par d'autres composants est déterminée en dotant la surface d'une molécule réceptrice.

**16.** Méthode selon l'une au moins des revendications précédentes,
**caractérisée en ce que**
un flux d'échantillon est mis en contact avec le détecteur de façon à déterminer la quantité de molécule cible en fonction du temps.

**17.** Méthode selon l'une au moins des revendications précédentes,
**caractérisée en ce que**
la quantité d'acides nucléiques contenant une certaine séquence est détectée et/ou déterminée dans un échantillon en utilisant une molécule réceptrice qui est un acide nucléique simple brin ayant une séquence complémentaire de celle de la molécule à détecter.

**18.** Méthode selon l'une au moins des revendications précédentes,
**caractérisée en ce que**
des mutations ponctuelles sont détectées dans un acide nucléique en mettant un échantillon en contact simultané avec deux ou plusieurs surfaces détectrices dotées de brins d'acides nucléiques récepteurs qui sont respectivement complémentaires de la séquence mutée et de la séquence normale, et la différence de leur réponse est déterminée.

Fig. 1

Fig. 2

Fig. 3

Fig. 4

Fig. 5